# EUROPÄISCHE PATENTSCHRIFT

(11) **EP 1 119 826 B1**
(45) Veröffentlichungstag und Bekanntmachung des Hinweises auf die Patenterteilung: **18.06.2003**
(21) Anmeldenummer: 99946019.9
(22) Anmeldetag: 24.08.1999
(51) Int. Cl.: G06M 1/04

(54) **ZÄHLWERK**
METER
DISPOSITIF COMPTEUR

(30) Priorität: 08.10.1998 DE 19846382
(43) Veröffentlichungstag der Anmeldung: 01.08.2001
(73) Patentinhaber: PARI GmbH Spezialisten für effektive Inhalation, 82319 Starnberg (DE)
(72) Erfinder: GALLEM, Thomas, D-81369 München (DE); STAPLETON, Kevin, Brookline, MA 02446-3011 (US); KNOCH, Martin, D-82335 Berg (DE)
(74) Vertreter: HOFFMANN - EITLE
(86) Internationale Anmeldenummer: EP9906206
(87) Internationale Veröffentlichungsnummer: WO00022570

(56) Entgegenhaltungen:
- WO-A-95/34874
- DE-B- 1 016 470
- GB-A- 1 317 315

## Beschreibung

Die vorliegende Erfindung betrifft ein Zählwerk, und insbesondere ein Zählwerk zur Verwendung mit Dosieraerosolvorrichtungen, wie beispielsweise Inhalatoren oder Vernebler.

Verschiedene Dosieraerosolvorrichtungen für Inhalationszwecke sind bekannt, die beispielsweise zur Behandlung von Atemwegserkrankungen eine dosisgenaue Medikamentenmenge in Form eines Sprühnebels oder Aerosols abgeben. Hierbei kommt es insbesondere auf die zuverlässige Dosierung des abgegebenen Medikaments an, damit ein gezielter Therapieeffekt beim Patienten hervorgerufen wird. Derartige Dosieraerosolvorrichtungen sind beispielsweise sogenannte MDI (metered-dose inhalers), die in der Regel einen Behälter zur Aufnahme eines flüssigen oder pulverförmigen Medikaments aufweisen, eine geeignet ausgebildete Düse zum Zerstäuben und Verteilen des Medikaments und eine Betätigungsvorrichtung mit einem Mundstück, über das das erzeugte medikamentenhaltige Aerosol eingeatmet wird. Die Betätigung der Dosieraerosolvorrichtungen erfolgt beispielsweise bei Treibgasaerosolen derart, daß der Medikamentenbehälter gegenüber der in der Betätigungsvorrichtung angeordneten Düse geradlinig verschoben wird, wodurch eine definierte Menge des Zerstäubungsguts freigesetzt wird. Eine Dosieraerosolvorrichtung dieser Art ist beispielsweise in EP 0 254 391 beschrieben.

Dosieraerosolvorrichtungen sind in der Regel für Mehrfachdosierungen ausgelegt. Dabei ist es wünschenswert, dem Benutzer die Anzahl der ausgegebenen Dosen, in anderen Worten der abgegebenen Sprühstöße, bzw. die noch zur Verfügung stehenden Sprühstöße anzuzeigen, um sicher zustellen, daß der Patient frühzeitig darauf hingewiesen wird, daß das Medikament verbraucht ist. Auf diese Weise wird vermieden, daß ein Patient, beispielsweise zur Vorbeugung eines akuten Asthmaanfalls, eine bereits nahezu leere Dosieraerosolvorrichtung mit sich führt.

Aus diesem Grund sind Dosiervernebler bzw. Dosierinhalatoren mit unterschiedlichen Zählwerken ausgestattet worden. EP 0 254 391 beschreibt zum Beispiel einen Inhalator mit einem flachen Zählwerk, das auf der dem Patienten zugewandten Seite eines Aerosolspenders ausgebildet ist. Aus EP 0 505 321 ist ein wiederverwendbarer Inhalator mit rücksetztbarem Zähler bekannt, der beim Erreichen der ersten Relativposition von Vorratskammer und Dosierstift inkrementiert wird. Das in GB 1 317 315 offenbarte Zählwerk weist eine Vielzahl von ringförmigen Elementen auf, die mechanisch zusammenwirken, um an einem Anzeigering die noch zur Verfügung stehenden Dosierungen anzuzeigen. Weitere Medikamentenspender mit mechanischem Zählwerk sind beispielsweise in WO 86/02275 und WO 93/24167 offenbart. WO 95/34874 beschreibt ein Zählwerk für Inhalatoren, das parallel zueinander liegende Zählscheiben aufweist.

Die bekannten Zählwerke für Dosierungsvorrichtungen weisen jedoch entweder einen komplexen, unhandlichen Aufbau auf oder erfordern, daß die bereits vorhandene Dosisaerosolvorrrichtung in erheblichem Umfang modifiziert werden muß, um zusammen mit einem Zählwerk verwendet werden zu können. Eine derartige Modifikation einer bereits vorhandenen Dosierungsvorrichtung ist jedoch insofern von Nachteil, da diese dann erneut einem amtlichen Genehmigungsverfahren und den damit einhergehenden Versuchen für die medizinische Anwendbarkeit unterworfen werden muß. Dies ist jedoch in der Regel ein langwieriger und kostspieliger Prozess und ist daher unerwünscht.

Überdies sind die bekannten Zählwerke aufwendig und eignen sich daher nicht zur Massenproduktion eines im übrigen sehr kostengünstig herzustellenden Produkts.

Ausgehend vom Stand der Technik ist es daher die Aufgabe der vorliegenden Erfindung, ein Zählwerk mit kleinen Abmessungen zu schaffen, das einen einfachen und somit kostengünstigen Aufbau aufweist, und das mit unterschiedlichen Inhalatoren zur Dosierungszählung verwendet werden kann, ohne daß wesentliche Modifikationen an den Inhalatoren durchgeführt werden müssen.

Die Aufgabe wird erfindungsgemäß durch ein Zählwerk gelöst mit vier Scheibeneinheiten, die parallel zueinander angeordnet sind, deren Mittelpunkte auf einer zu den Ebenen der vier Scheibeneinheiten senkrecht stehenden Achse liegen und von denen eine erste Scheibeneinheit erste Aufnahmemittel zur Aufnahme einer eine Drehbewegung der ersten Scheibeneinheit um die Achse bewirkenden Kraft und erste Übertragungsmittel zur Übertragung der Drehbewegung auf eine zweite Scheibeneinheit aufweist, die zweite Aufnahmemittel zur Aufnahme einer durch die ersten Übertragungsmittel übertragenen und eine Drehbewegung der zweiten Scheibeneinheit um die Achse bewirkenden Kraft und zweite Übertragungsmittel zur Übertragung der Drehbewegung auf eine vierte Scheibeneinheit aufweist, die dritte Aufnahmemittel zur Aufnahme einer durch die zweiten Übertragungsmittel übertragenen und eine Drehbewegung der vierten Scheibeneinheit um die Achse bewirkenden Kraft aufweist.

Vorteilhaft definiert die dritte Scheibeneinheit eine feste Position, zu der die erste, zweite und vierte Scheibeneinheiten drehbar gelagert sind.

In einem Ausführungsbeispiel sind die ersten Aufnahmemittel der ersten Scheibeneinheit eine Außenverzahnung. Ferner sind die ersten Übertragungsmittel und die zweiten Aufnahmemittel in Form einer festen Verbingung zwischen der ersten und der zweiten Scheibeneinheit realisiert.

In dem Ausführungsbeispiel sind die zweiten Übertragungsmittel in Form eines Federnocken, der an der Außenkontur der zweiten Scheibeneinheit federnd angeordnet ist, und die dritten Aufnahmemittel in Form einer Innenverzahnung an der vierten Scheibeneinheit realisiert.

Damit der Federnocken mit der Innenverzahnung der vierten Scheibeneinheit in Eingriff gelangt, ist an der Außenkontur der dritten Scheibeneinheit mindestens ein Vorsprung vorgesehen, der bei einer vorbestimmten Drehstellung der zweiten Scheibeneinheit in Bezug auf die dritte Scheibeneinheit den Federnocken in radialer Richtung auslenkt.

In einer vorteilhaften Ausgestaltung ist der Federnocken keilförmig, und die Breite des Federnockens ist größer als die Dicke der zweiten Scheibeneinheit, so daß der Federnocken die zweite Scheibeneinheit in Richtung der Achse überragt.

Um ein unbeabsichtigtes Rückstellen des Zählwerks zu vermeiden, weist die vierte Scheibeneinheit eine um den Mittelpunkt der vierten Scheibeneinheit kreisförmig ausgebildete zweite Verzahnung und die dritte Scheibeneinheit eine Vielzahl von Rastverzahnungen für den Eingriff in die zweite Verzahnung der vierten Scheibeneinheit auf.

Vorteilhaft ist der Radius der zweiten Scheibeneinheit größer als der der ersten Scheibeneinheit, der Radius der dritten Scheibeneinheit ist größer als der der ersten Scheibeneinheit und kleiner als der der zweiten Scheibeneinheit, und der Radius der vierten Scheibeneinheit ist größer als der der ersten, zweiten und dritten Scheibeneinheit.

Zur Lagerung der vier Scheibeneinheiten ist in einer bevorzugten Ausführungsform ein Lagerbolzen vorgesehen, der einen ersten Abschnitt für die gleitende Lagerung der ersten und zweiten Scheibeneinheit und einen zweiten Abschnitt für die gleitende Lagerung der vierten Scheibeneinheit aufweist und an dem die dritte Scheibeneinheit zwischen dem ersten und zweiten Abschnitt befestigt ist.

Der Lagerbolzen dient vorzugsweise auch zur Befestigung des Zählwerks an einem Gegenstand, beispielsweise einem Inhalator oder Dosieraerosolerzeuger, und besitzt dazu einen dritten Abschnitt, der sich auf der Seite der ersten Scheibeneinheit aus dem Zählwerk heraus erstreckt.

Zur Sicherung gegen eine unbeabsichtigte Rückstellung weist die zweite Scheibeneinheit eine Innenverzahnung auf. Als Gegenstücke sind an dem Lagerbolzen Verriegelungselemente, vorzugsweise L-förmig, befestigt, die mit der Innenverzahnung der zweiten Scheibeneinheit in Wechselwirkung stehen.

In einer besonders vorteilhaften Ausgestaltung umfasst das Zählwerk vier Scheibeneinheiten, die parallel zueinander angeordnet sind, deren Mittelpunkte auf einer zu den Ebenen der vier Scheibeneinheiten senkrecht stehenden Achse liegen. Eine erste Scheibeneinheit besitzt erste Aufnahmemittel zur Aufnahme einer eine Drehbewegung der ersten Scheibeneinheit um die Achse bewirkenden Kraft und erste Übertragungsmittel zur Übertragung der Drehbewegung auf eine zweite Scheibeneinheit. Die zweite Scheibeneinheit weist zweite Aufnahmemittel zur Aufnahme einer durch die ersten Übertragungsmittel übertragenen und eine Drehbewegung der zweiten Scheibeneinheit um die Achse bewirkenden Kraft auf. Ferner weist die zweite Scheibeneinheit einen Federnocken auf, der an der Außenkontur der zweiten Scheibeneinheit federnd angeordnet ist, zur Übertragung der Drehbewegung auf eine vierte Scheibeneinheit, die wiederum eine Innenverzahnung zur Aufnahme einer durch den Federnocken übertragenen und eine Drehbewegung der vierten Scheibeneinheit um die Achse bewirkenden Kraft besitzt. Dabei ist an der Außenkontur einer dritten Scheibeneinheit mindestens ein Vorsprung vorgesehen, der bei einer vorbestimmten Drehstellung der zweiten Scheibeneinheit in Bezug auf die dritte Scheibeneinheit den Federnocken in radialer Richtung auslenkt, so daß der Federnocken in Eingriff mit der Innenverzahnung der vierten Scheibeneinheit gerät, wobei mindestens ein Zahn der Innenverzahnung der vierten Scheibeneinheit ausgefüllt ist, um einen Verriegelungseffekt zu bewirken. Die Vorteile dieser Ausgestaltung werden bei der Beschreibung der Ausführungsbeispiele dargestellt.

In einer alternativen, aber ebenfalls besonders vorteilhaften Ausgestaltung umfasst das Zählwerk vier Scheibeneinheiten, die parallel zueinander angeordnet sind, deren Mittelpunkte auf einer zu den Ebenen der vier Scheibeneinheiten senkrecht stehenden Achse liegen. Eine erste Scheibeneinheit besitzt erste Aufnahmemittel zur Aufnahme einer eine Drehbewegung der ersten Scheibeneinheit um die Achse bewirkenden Kraft und erste Übertragungsmittel zur Übertragung der Drehbewegung auf eine zweite Scheibeneinheit. Die zweite Scheibeneinheit weist zweite Aufnahmemittel zur Aufnahme einer durch die ersten Übertragungsmittel übertragenen und eine Drehbewegung der zweiten Scheibeneinheit um die Achse bewirkenden Kraft auf. Ferner weist die zweite Scheibeneinheit einen Federnocken auf, der an der Außenkontur der zweiten Scheibeneinheit federnd angeordnet ist, zur Übertragung der Drehbewegung auf eine vierte Scheibeneinheit, die wiederum eine Innenverzahnung zur Aufnahme einer durch den Federnocken übertragenen und eine Drehbewegung der vierten Scheibeneinheit um die Achse bewirkenden Kraft besitzt. Dabei ist an der Außenkontur einer dritten Scheibeneinheit mindestens ein Vorsprung vorgesehen, der bei einer vorbestimmten Drehstellung der zweiten Scheibeneinheit in. Bezug auf die dritte Scheibeneinheit den Federnocken in radialer Richtung auslenkt, so daß der Federnocken in Eingriff mit der Innenverzahnung der vierten Scheibeneinheit gerät, wobei mindestens ein Zahn der Innenverzahnung der vierten Scheibeneinheit entfernt ist, um einen Eingriff des Federnockens zu unterdrücken. Die Vorteile dieser Ausgestaltung werden bei der Beschreibung der Ausführungsbeispiele dargestellt.

Eine besondere Verwendung findet das erfindungsgemäße Zählwerks der oben beschriebenen Art in Inhalatoren, Verneblern oder ähnlichen Dosieraerosolvorrichtungen. Für diesen Zweck ist das Zählwerk besonders ausgelegt und eignet sich aufgrund der einfachen und damit kostengünstigen Gestaltung hervorragend für diesen Einsatz. Insbesondere die Befestigung mittels des aus dem Zählwerk herausragenden Endes des Lagerbolzens trägt zu der problemlosen Verwendung in einer beliebigen Dosieraerosolvorrichtung bei.

Dabei handelt es sich um einen wesentlicher Vorteil der Erfindung, da das erfindungsgemäße Zählwerk mit verschiedenen Inhalatoren, Verneblern oder gleichartigen Dosieraerosolvorrichtungen verwendet werden kann, ohne daß die bekannten Vorrichtungen wesentlich modifiziert werden müssen. Dies hat den Vorteil, daß die Dosieraerosolvorrichtungen nicht erneut zeitraubenden und kostspieligen Versuchen für die klinische Anwendbarkeit und Genehmigungsverfahren unterworfen werden müssen.

Zudem weist das erfindungsgemäße Zählwerk kleine Abmessungen auf und kann somit platzsparend zusammen mit der Dosiervorrichtung als handliches System ausgebildet werden.

Weitere Merkmale und Vorteile der vorliegenden Erfindung ergeben sich aus der folgenden Beschreibung eines bevorzugten Ausführungsbeispieles, das im Zusammenhang mit den beigefügten Abbildungen erläutert wird, in denen zeigt:
- Fig. 1: eine Explosionsdarstellung des erfindungsgemäßen Zählwerks, das mit einer Betätigungseinheit eines Dosieraerosolerzeugers verbunden ist;
- Fig. 2: eine Explosionsdarstellung des in Fig. 1 gezeigten Zählwerks, betrachtet aus der zu Fig. 1 anderen Blickrichtung;
- Fig. 3: eine schematische Querschnittsansicht einer Ausgangs- bzw. Ruheposition der zweiten und dritten Scheibeneinheiten zur Erläuterung des Zusammenwirkens von zweiter und dritter Scheibeneinheit;
- Fig. 4: eine schematische Querschnittsansicht analog zu Fig.3, die die zweite und dritte Scheibeneinheit in einer Position zeigt, in der die zweite Scheibeneinheit durch die dritte Scheibeneinheit veranlaßt wird, mit der vierten Scheibeneinheit zusammenzuwirken;
- Fig. 5A und 5B: jeweils einen Auschnitt der Innenverzahnung der vierten Scheibeneinheit mit einer Zählwertbegrenzung;
- Fig. 6: eine Seitenansicht einer alternativen Ausgestaltung der in Fig. 1 und 2 dargestellten Betätigungseinheit; und
- Fig. 7: ein Verwendungsbeispiel des erfindungsgemäßen Zählwerks in einer Dosieraerosolvorrichtung.

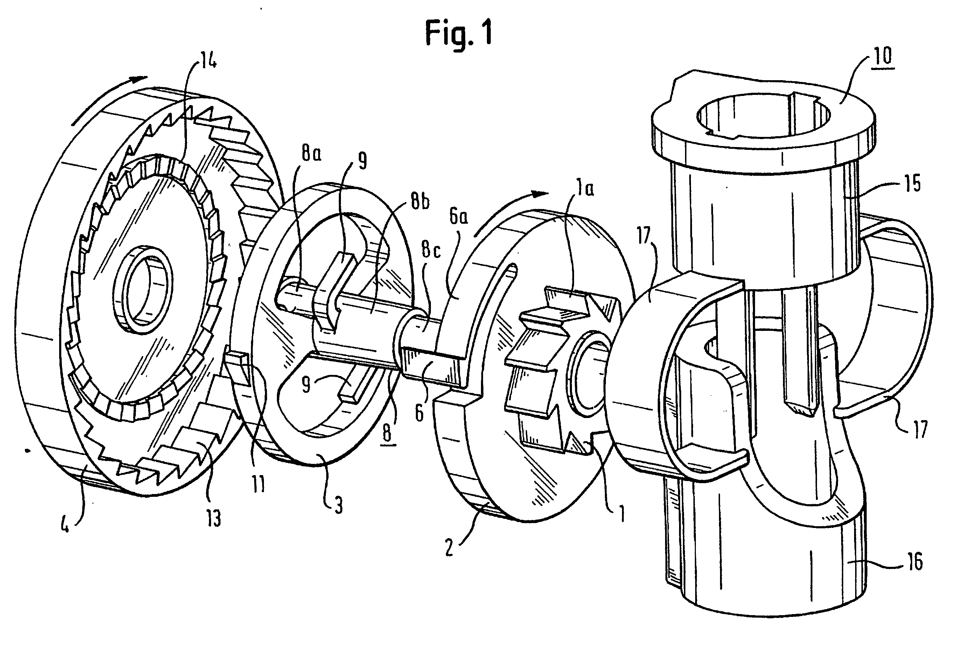

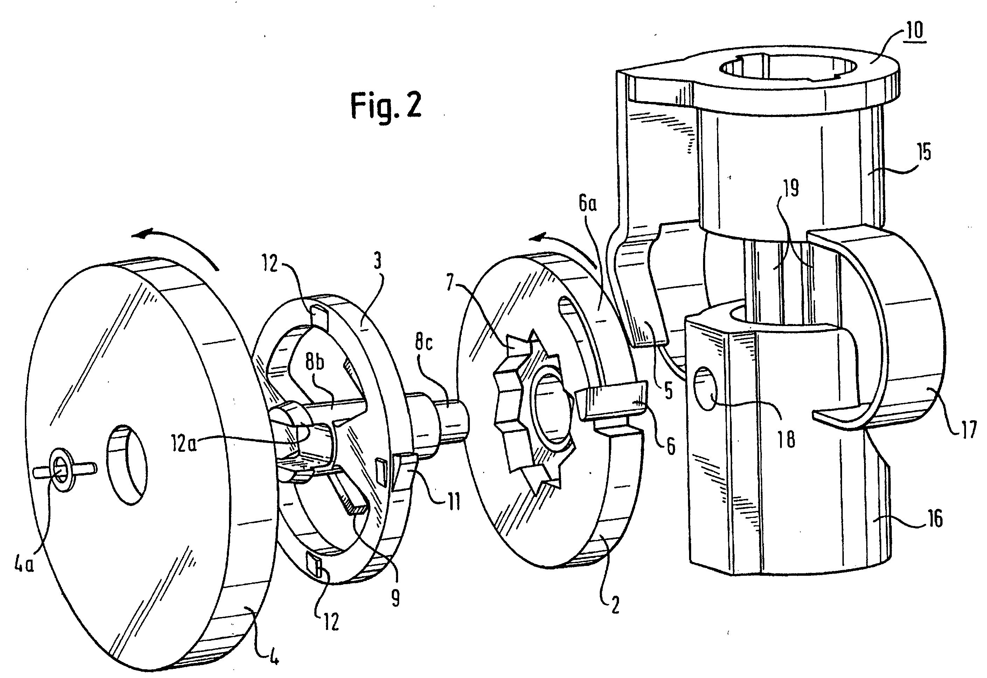

Die einzelnen Komponenten des erfindungsgemäßen Zählwerks werden im folgenden unter Bezugnahme auf Fig. 1 und 2 beschrieben. Fig. 1 und 2 zeigen jeweils aus zwei unterschiedlichen Blickrichtungen betrachtete Explosionsdarstellungen des Zählwerks.

Das Zählwerk weist im wesentlichen vier Scheibeneinheiten 1, 2, 3 und 4 auf, die parallel zueinander angeordnet sind. Die Mittelpunkte der Scheibeneinheiten 1, 2, 3 und 4 liegen auf einer zu den Scheibeneinheiten senkrecht stehenden Achse und sind, wie nachstehend noch genauer erläutert wird, über einen Lagerbolzen 8 mit einer Betätigungseinheit 10 eines Dosieraerosolerzeugers (nicht dargestellt) verbunden. Die erste, zweite und vierte Scheibeneinheit 1, 2 und 4 sind bezogen auf die Betätigungseinheit 10 drehbar angeordnet. Die dritte Scheibeneinheit 3 nimmt eine feste Position bezogen auf die Betätigungseinheit 10 und damit auch auf die übrigen Scheibeneinheiten ein.

Die erste Scheibeneinheit 1 besitzt ein Mittel zur Aufnahme einer Kraft, die eine Drehung der ersten Scheibeneinheit hervorruft; dabei handelt es sich um eine Außenverzahnung 1a, in die ein an der Betätigungseinheit 10 angebrachter Betätigungsarm 5 eingreift (siehe Fig. 1 und 2). Die erste Scheibeneinheit 1 weist einen kleineren Radius als die zweite Scheibeneinheit 2 auf und ist mit dieser fest verbunden. Durch diese feste Verbindung wird die Übertragung der Drehbewegung von der ersten Scheibeneinheit 1 auf die zweite Scheibeneinheit 2 realisiert. Es können aber auch andere Mittel zur Übertragung der Drehbewegung und zur Aufnahme der eine Drehbewegung hervorufenden Kraft an den beiden Scheibeneinheiten vorgesehen sein.

Um die Drehbewegung an eine vierte Scheibeneinheit 4 zu übertragen, ist als Übertragungsmittel am Außenumfang der zweiten Scheibeneinheit 2 ein keilförmiger Nocken 6 angebracht, der über ein Federelement 6a mit der zweiten Scheibeneinheit 2 verbunden ist und der im folgenden als Federnocken bezeichnet wird. Das Federelement 6a bildet im wesentlichen die Außenkontur der zweiten Scheibeneinheit 2 nach, besitzt aber ein frei schwingendes Ende. An dem frei schwingendem Ende ist der keilförmige Federnocken 6 derart angebracht, daß er in der Ruhestellung über die Außenkontur der zweiten Scheibeneinheit 2 in radialer Richtung übersteht. Die Geometrie des Federnocken ist so gewählt, daß der Nocken bzw. die Nase bei einer Auslenkung in radialer Richtung in die Innenverzahnung 13 der vierten Scheibeneinheit 4 eingreift.

Dazu ist der keilförmige Federnocken 6 so angeordnet, daß die scharfe und die stumpfe Kante senkrecht zur Ebene der zweiten Scheibeneinheit 2 orientiert sind. Der Federnocken 6 weist ferner eine Breite auf, die größer ist als die Dicke der zweiten Scheibeneinheit 2, so daß sich der Federnocken 6 parallel zur Achse der Scheibeneinheiten zapfenartig in Richtung auf die dritte und vierte Scheibeneinheit 3 und 4 erstreckt.

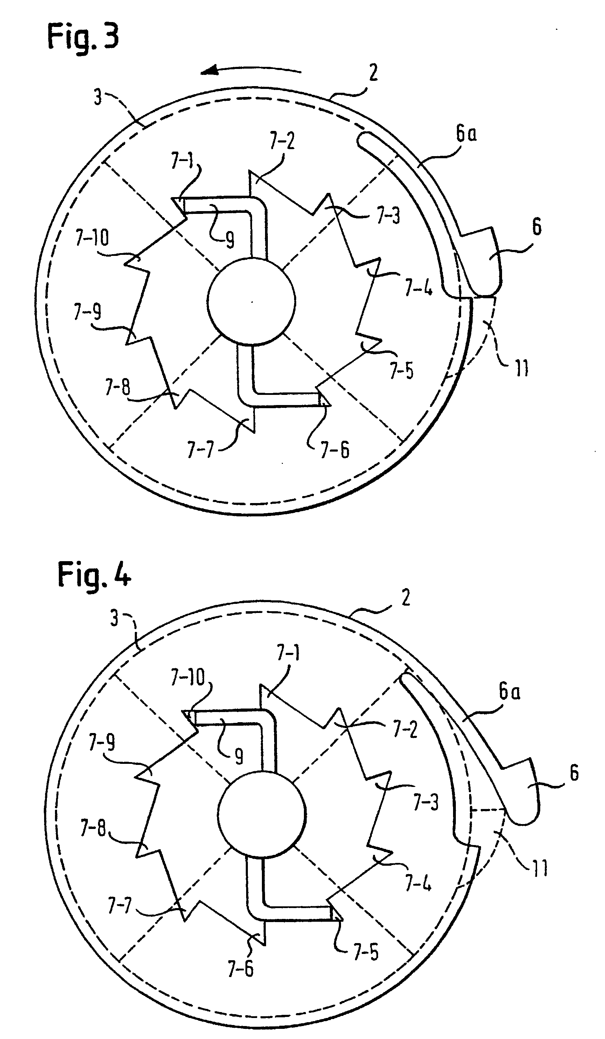

Der zapfenartig in Richtung dritte und vierte Scheibeneinheit überstehenden Federnocken 6 kann in Wechselwirkung mit den anderen Elementen des Zählwerks, die insbesondere unter Bezugnahme auf Fig. 3 und 4 genauer erläutert werden, mit einer ersten Verzahnung 13 der vierten Scheibeneinheit 4 in Eingriff treten (siehe Fig. 2).

Die in Richtung dritte und vierte Scheibeneinheit weisende Seite der zweiten Scheibeneinheit 2 weist eine Innenverzahnung 7 auf, die symmetrisch um die Achse der Scheibeneinheiten angeordnet ist. Die Innenverzahnung 7, die in Fig. 2 zu sehen ist, nicht aber in Fig.1, weist die gleiche Anzahl von Zähnen wie die außenverzahnte Scheibeneinheit 1 auf. In der in Fig. 1 bzw. 2 dargestellten Ausführungsform beträgt die Anzahl der Zähne zehn, um zusammen mit einer ersten Innenverzahnung 13 der vierten Scheibeneinheit 4 mit 24 Zähnen insgesamt 240 diskrete Zählschritte zu realisieren. Jedoch können problemlos andere Werte verwendet werden.

Der Radius der dritten Scheibeneinheit 3 ist gleich oder vorzugsweise geringfügig kleiner als der Radius der zweiten Scheibeneinheit 2. Ferner ist an dem Außenumfang der dritten Scheibeneinheit 3 eine keilförmige Schulter 11 angebracht, die zur Betätigung des an der zweiten Scheibeneinheit 2 angeordneten Federnocken 6 dient, was im folgenden noch genauer erklärt wird. Wird eine weitere Schulter angebracht, so ändert sich das Übersetztungsverhältnis. Die dritte Scheibeneinheit 3 ist mit einem Lagerbolzen 8 fest verbunden.

Der Lagerbolzen 8 weist bezogen auf die dritte Scheibeneinheit 3 drei unterschiedliche Bereiche auf, nämlich den in Richtung vierte Scheibeneinheit 4 weisenden Abschnitt 8a und die in Richtung erste und zweite Scheibeneinheit weisenden Abschnitte 8b und 8c. Der Durchmesser des Lagerbolzenabschnitts 8b ist mit dem des Abschnittes 8a identisch, und der des Abschnitts 8c ist im Vergleich dazu reduziert. Der Lagerbolzen 8 dient der drehfesten Anbringung der dritten Scheibeneinheit 3 an der Betätigungseinheit 10 und zur drehbaren Lagerung der ersten, zweiten und vierten Scheibeneinheit 1, 2 und 4. Hierzu ist der Lagerbolzenabschnitt mit kleinerem Radius 8c in eine entsprechende Öffnung 18 der Befestigungseinheit 10 eingeführt und derart befestigt, daß die Position der dritten Scheibeneinheit 3 bezogen auf die Betätigungseinheit 10 fest ist. Die in der ersten und zweiten Scheibeneinheiten 1 und 2 vorgesehenen Öffnungen sind so auf den Durchmesser des Lagerbolzenabschnittes 8b abgestimmt, daß die ersten und zweiten Scheibeneinheiten 1 und 2 auf dem Lagerbolzenabschnitt 8b gleitend drehbar sind. Es sei angemerkt, daß die Durchmesser der Abschnitte 8a, 8b und 8c nicht auf das in Fig. 1 bzw. Fig. 2 dargestellte Verhältnis beschränkt sind. Wesentlich ist, daß die jeweiligen Öffnungen der Scheibeneinheiten und die Durchmesser der Lagerbolzenabschnitte aufeinander abgestimmt sind.

Ferner sind an dem Lagerbolzen 8 zwei L-förmige Verriegelungselemente 9 angeordnet, die am Außenumfang des Lagerbolzenabschnitts 8b angeordnet sind und die in die Innenverzahnung 7 der zweiten Scheibeneinheit 2 eingreifen. Die Öffnung der vierten Scheibeneinheit 4 ist so auf den Durchmesser des Lagerbolzenabschnittes 8a abgestimmt, daß die vierte Scheibeneinheit 4 auf dem Lagerbolzenabschnitt 8a gleitend drehbar ist. An dem Abschnitt 8a sind zudem, in Richtung vierte Scheibeneinheit 4 weisend, zwei Schnapphaken 12a symmetrisch angeordnet, die zur axialen Fixierung der Scheibeneinheit 4 dienen.

Auf der in Richtung vierte Scheibeneinheit 4 weisenden Seite der dritten Scheibeneinheit 3 ist eine Rastverzahnung 12 angebracht, die in Fig. 2 zu sehen ist. In dem in Fig. 2 dargestellten Ausführungsbeispiel besteht die Rastverzahnung 12 aus vier Elementen, die um 90° zueinander beabstandet sind. Die Rastverzahnung kann aber auch aus einem oder aus mehreren Elementen bestehen, die zentrisch um die Achse des Lagerbolzens 8 angeordnet sind. Diese Rastverzahnung 12 greift in eine zweite Verzahnung 14 der vierten Scheibeneinheit 4 ein und dient im wesentlichen der Sicherung der Drehstellung der Scheibeneinheit 4.

Die Ausgestaltung der vierten Scheibeneinheit 4 ist insbesondere aus Fig. 1 ersichtlich. Die in Richtung der anderen Scheibeneinheiten weisende Seite der vierten Scheibeneinheit 4 weist eine erste Verzahnung 13 auf, die an der Umfangsinnenseite angeordnet ist, und eine zweite Verzahnung 14, die kreisförmig um den Mittelpunkt der vierten Scheibeneinheit 4 verläuft. Der Radius der zweiten Verzahnung 14 ist abgestimmt auf die Lage der Elemente der Rastverzahnung 12, die an der dritten Scheibeneinheit 3 vorgesehen ist.

Im montierten Zustand sind die erste und zweite Scheibeneinheit 1 und 2 auf dem Lagerbolzenabschnitt 8b angeordnet; dabei liegen die L-förmigen Verrigerlungselemente 9 in der Ausnehmung der Innenverzahnung 7 der zweiten Scheibeneinheit 2. Der Federnocken 6 ragt über die Außenkontur der dritten Scheibeneinheit 3. Die vierte Scheibeneinheit 4 ist auf dem Lagerbolzenabschnitt 8a angeordnet und nimmt aufgrund der kappenartigen Gestalt die dritte Scheibeneinheit 3 auf; dabei liegt der Federnocken 6 zwischen der Außenkontur der dritten Scheibeneinheit 3 und der ersten Innenverzahnung der vierten Scheibeneinheit 4.

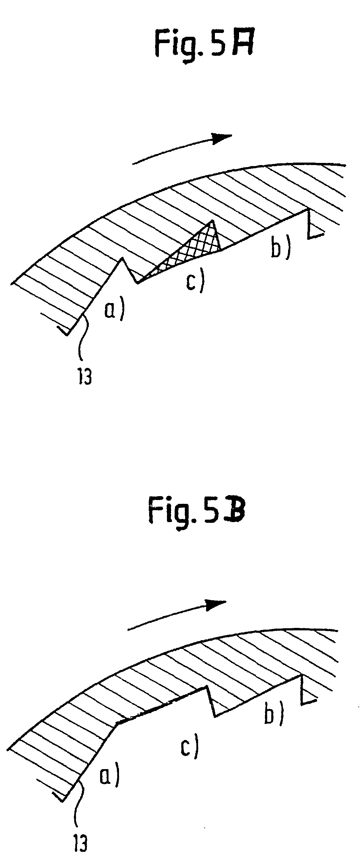

Im folgenden wird die Funktionsweise des erfindungsgemäßen Zählwerks beschrieben. Da, wie in Fig. 2 gezeigt, ein Betätigungsarm 5 in die Außenverzahnung der ersten Scheibeneinheit 1 eingreift, wird durch eine Betätigung des Armes 5, was unter Bezugnahme auf Fig. 5 nachstehend noch genauer beschrieben wird, die erste Scheibeneinheit 1 in die in Fig. 1 und 2 angedeutete Pfeilrichtung gedreht. Hierbei bewegt sich die zweite Scheibeneinheit 2 mit, da die erste und zweite Scheibeneinheit 1 und 2 fest miteinander verbunden sind. Die erste und zweite Scheibeneinheit 1 und 2 gleiten dabei auf dem Lagerbolzenabschnitt 8b. Im Gegensatz dazu ist die dritte Scheibeneinheit 3 über den Lagerbolzen 8 mit der Betätigungseinrichtung 10 fest verbunden, so daß die Position der dritten Scheibeneinheit 3 bezogen auf die Betätigungseinrichtung 10 zu jedem Zeitpunkt unverändert bleibt. Gleichzeitig sind die an dem Lagerbolzenabschnitt 8b angebrachten L-förmigen Verriegelungselemente 9 in Eingriff mit der Innenverzahnung 7 der Scheibeneinheit 2; jedoch gestattet die Gestaltung der Verriegelungselemente 9 und der Innenverzahnung 7 eine Drehung der ersten und zweiten Scheibeneinheiten 1 und 2 in der in Fig. 1 und 2 gezeigten Richtung. Bei einer Drehung der ersten und zweiten Scheibeneinheit 1 und 2 in Pfeilrichtung rasten die Verriegelungselemente 9 in die jeweils nächste Verzahnung der Innenverzahnung 7 ein. Bei einer erneuten Betätigung des Armes 5 drehen sich Scheibeneinheiten 1 un 2 genau einen Zahn weiter und der Verriegelungsarm rastet in den nächsten Zahn ein. Durch ein derartiges Eingreifen der Verriegelungselemente 9 in die Innenverzahnung 7 wird sichergestellt, daß sich die erste und somit auch die zweite Scheibeneinheit beim Lösen des Betätigungsarms 5 aus dem Eingriff mit der zahnradförmigen Scheibeneinheit 1 nicht zurückdreht, d.h. sich nicht entgegen der Pfeilrichtung zurückbewegt.

Bei der in Fig. 1 und 2 angedeueteten Drehung der ersten und zweiten Scheibeneinheit 1 und 2 in Pfeilrichtung läuft der an der zweiten Scheibeneinheit 2 angeordnete Federnocken 6 auf der Außenkontur der dritten Scheibeneinheit 3 entlang. Geht man von einem Zahnrad, das zehn Zähne aufweist aus, so überstreicht der Federnocken 6 bei 10-maliger Betätigung des Armes 5 einen Winkel von 360°, d.h. der Federnocken macht einen vollen Umlauf und gelangt wieder an seine Ausgangsposition. Solange der Federnocken 6 nicht von der Schulter 11 der dritten Scheibeneinheit 3 beeinflußt wird, greift der Federnocken 6 nicht in die an der Innenumfangseite der vierten Scheibeneinheit 4 angebrachte erste Verzahnung 13 ein. Erst wenn die Schulter 11 den Federnocken 6 in radialer Richtung anhebt, gelangt der Federnocken 6 in Eingriff mit der ersten Innenverzahnung der vierten Scheibeneinheit 4 und bewirkt eine Drehung der vierten Scheibeneinheit um eine Drehstellung.

Die schrittweise Drehung der Scheibeneinheit 2 bezogen auf die dritte Scheibeneinheit 3, sowie das Eingreifen des Federnockens 6 der zweiten Scheibeneinheit 2 in die Innenverzahnung 13 der vierten Scheibeneinheit 4 werden im folgenden unter Bezugnahme auf Fig. 3 und 4 genauer beschrieben. Fig. 3 und 4 zeigen schematische Querschnittsansichten, die insbesondere das Zusammenwirken von zweiter und dritter Scheibeneinheit 2 und 3 detaillierter erläutern. In Fig. 3 und 4 sind die Scheibeneinheiten 2 und 3 aus der in Fig. 2 gezeigten Blickrichtung dargestellt. Die dritte Scheibeneinheit 3 ist in beiden Abbildungen aus Gründen der besseren Übersicht lediglich gestrichelt angedeutet. Fig. 3 zeigt die oben erwähnte Ausgangsposition bzw. Ruheposition der zweiten Scheibeneinheit 2 in Bezug auf die fest mit der Betätigungseinrichtung 10 verbundene dritte Scheibeneinheit 3. In dieser Position ist das Verriegelungselement 9 in den ersten Zahn 7-1 der Innenverzahnung 7 eingerastet. Durch Betätigung des Betätigungsarmes 5, der in die zahnradförmige erste Scheibeneinheit 1 eingreift (in Fig. 3 nicht dargestellt), wird die Scheibeneinheit 2 schrittweise, d.h. Zahn für Zahn, in der in Fig. 3 angedeuteten Pfeilrichtung gedreht. Das Verriegelungselement 9 rastet hierbei, beginnend vom ersten Innenzahn 7-1 in aufsteigender Reihenfolge in die Zähne 7-2 bis 7-9 ein. Bei der durch den Betätigungsarm 5 initiierten Drehbewegung der ersten und zweiten Scheibeneinheit 1 und 2 läuft der Federnocken 6 auf dem Umfang der in Fig. 3 gestrichelt dargestellten dritten Scheibeneinheit 3 entlang, ohne daß der Federnocken 6 ausgelenkt wird. In diesem Fall greift der Federnocken 6 nicht in die erste Verzahnung 13 der vierten Scheibeneinheit 4 ein.

Ist der Betätigungsarm 5 so oft betätigt worden, daß das Verriegelungselement 9 in den Zahn 7-10 der Innenverzahnung 7 einrastet, d.h wenn ausgehend von der in Fig. 3 dargestellten Ausgangsposition der Betätigungsarm 5 zum neunten Mal betätigt worden ist, dann ist der Federnocken 6 in Kontakt mit der Schulter 11. Dies bewirkt, daß der Federnocken 6, wie in Fig. 4 dargestellt, in radialer Richtung ausgelenkt wird. Mit anderen Worten, der Federnocken 6 wird in dieser Position durch die Schulter 11 betätigt und der Federnocken 6 greift in die Innenumfangsverzahnung 13 ein und dreht die vierte Scheibeneinheit 4 um einen Zahn der ersten Verzahnung 13 in Pfeilrichtung weiter.

Bei dieser durch das Zusammenwirken der Schulter 11 und des Federnocken 6 bewirkten Drehung der vierten Scheibeneinheit 4 rastet die an der in Richtung erste, zweite und dritte Scheibeneinheit weisenden Innenseite der vierten Scheibeneinheit 4 angeordnete zweite Verzahnung 14 mit der Rastverzahnung 12 ein. Die Rastverzahnung 12 und die zweite Verzahnung 14 der vierten Scheibeneinheit 4 dienen hierbei der Sicherung der vierten Scheibeneinheit 4 gegen Verdrehen, und durch die Schnapphaken 12a ist eine axiale Fixierung der vierten Scheibeneinheit 4 gewährleistet.

Ist eine Verriegelung nach einmaligem Durchlauf aller Zähne der Scheibeneinheit 4 erwünscht, so kann diese dadurch erreicht werden, indem man statt wie beschrieben 24 Zähne nur 23 Zähne ausbildet und eine Zahnlücke frei lässt. Diese Art der Verriegelung ist in Fig. 5A dargestellt, die einen Ausschnitt der Innenverzahnung 13 der vierten Scheibeneinheit 4 zeigt. Die Drehung der vierten Scheibeneinheit 4 erfolgt in Pfeilrichtungen und die Positionen a)und b) deuten die Ausgangs- bzw. Endstellung an. Der dazwischen liegende Zahn (Position c)) ist nicht ausgebildet wodurch der Verriegelungseffekt erzielt wird.

In Fig. 5B ist eine weitere Ausgestaltung der vierten Scheibeneinheit 4 dargestellt, durch die verhindert wird, daß sich die vierte Scheibeneinheit 4 nach dem Erreichen eines maximalen Zählwerts weiterdreht. Bei diesem Ausführungsbeispiel ist an der Stelle c) ein Zahn der Innenverzahnung ausgelassen worden, so daß der Federnocken 6 auch in der durch die Schulter 11 ausgelenkten Stellung nicht mit der Innenverzahnung 13 der vierten Scheibeneinheit 4 in Eingriff gerät. Das bedeutet, daß nach einer nahezu vollständigen Umdrehung der vierten Scheibeneinheit 4 eine weitere Drehung verhindert wird.

Die Ausgestaltung gemäß Fig. 5B ist insofern vorteilhaft, als daß einerseits eine Weiterschaltung der vierten Scheibeneinheit 4 vermieden wird, jedoch andererseits eine weitere Bewegung der Betätigungseinrichtung und damit eine Betätigung des Verneblers (MDI) nicht beeinträchtigt wird. Für den Benutzer bedeutet dies, daß er zuverlässig angezeigt bekommt, wenn die maximale Sprühstoßzahl erreicht wurde, ohne daß er aber einer weiteren Benutzung des Verneblers (MDI) gehindert ist. Im Hinblick auf die mit Vernebler der hier in Rede stehenden Art (MDI) zu therapierenden Erkrankungen, beispielsweise Asthmaanfälle, ist dies ein wichtiger Vorteil, da durch die zweite Ausgestaltung gemäß Fig. 5B keine Verriegelung des Gesamtsystems erfolgt, die den Benutzer an der Abgabe eines - vielleicht lebensrettenden - Sprühstoßes hindert. Die erfindungsgemäße Gestaltung läßt die weitere Benutzung zu.

Tatsächlich ist zu erwarten, daß nicht nur in Notsituationen, wie oben beschrieben, sondern auch bei normalem Gebrauch der Benutzer auf die Vorrichtung mit einer so großen Kraft einwirkt, daß die Mechanik, die eigentlich eine Verriegelung bewirken soll, den einwirkenden Kräften nicht standhält und zumindest teilweise zerstört wird. Die sich dabei lösenden Teile werden als Bruchstücke in einem Moment erzeugt, in dem der Benutzer das entstehende Aerosol tief inhalieren will. Dabei können Partikel des zerstörten Zählwerks mit eingeatmet werden. Auch dies wird durch die Gestaltung gem. Fig. 5B wirksam vermieden, da eine weitere Benutzung der Vorrichtung möglich ist, ohne daß jedoch das Zählwerk sichtbar weitergeschaltet wird.

Bei der nachfolgenden Betätigung der ersten bzw. zweiten Scheibeneinheit durch den Betätigungsarm 5 nimmt die zweite Scheibeneinheit 2 wieder die in Fig. 3 dargestellte Ausgangsposition ein und die zuvor unter Bezugnahme auf Fig. 3 beschriebene Bewegung des Federnocken 6 wird wieder durchlaufen, ohne daß der Federnocken 6 in Eingriff mit der ersten Verzahnung 13 der vierten Scheibeneinheit 4 tritt. Erst nachdem das Verriegelungselement wieder in den Zahn 7-10 der Innenverzahnung 7 einrastet, wird der Federnocken 6 durch die Schulter 11 erneut ausgelenkt, so daß dieser wieder in Eingriff mit der Innenverzahnung 13 tritt und daraufhin die vierte Scheibeneinheit 4 um einen Zahn der ersten Innenverzahnung 13 weiterdreht. Auf diese Weise wird bei einer 10-zahnigen ersten Scheibeneinheit die vierte Scheibeneinheit 4 jeweils nach 10 Betätigungen des Betätigungsarms 5 um genau einen Zahn der ersten Verzahnung 13 weitergedreht. Wenn die erste Innenverzahnung 13 der vierten Scheibeneinheit 4 beispielsweise 24 Zähne aufweist, beträgt die maximale Anzahl der Zählstellungen in diesem Fall 240, d.h. wenn sich die vierte Scheibeneinheit 4 um 360° gedreht hat, sind 240 Betätigungen des Betätigungsarms 5 gezählt worden. Die zweite Verzahnung 14 der vierten Scheibeneinheit 4 weist die gleiche Anzahl von Zähnen wie die erste Innenverzahnung 13 auf.

Es sei angemerkt, daß die Anzahl der Zähne der ersten und zweiten Verzahnung 13 und 14 entsprechend an die gewünschte Übersetzung angepaßt werden kann. Eine 30-zahnige erste bzw. zweite Verzahnung 13 bzw. 14 dient somit zur Zählung von bis zu 300 Betätigungen. Das Übersetzungsverhältnis des erfindungsgemäßen Zählwerks bzw. die Anzahl der Zählungen kann aber auch durch ein Anordnen einer weiteren Schulter bzw. mehrerer Schultern am Außenrand der dritten Scheibeneinheit 3 variiert werden. Dies ermöglicht insbesondere ein unkompliziertes und schnelles Anpassen des erfindungsgemäßen Zählwerks an ein gewünschtes Übersetzungsverhältnis.

Die vierte Scheibeneinheit 4 kann in Form einer Anzeigescheibe ausgebildet sein, um entsprechend die Zählungen anzuzeigen. Dies kann beispielsweise durch einen Aufdruck eines Zeigers oder einer Farbmarkierung sowohl auf der Stirnseite als auch auf dem Rand der vierten Scheibeneinheit 4 erreicht werden. In dem in Fig. 2 dargestellten Ausführungsbeispiel ist ein Zeiger-Anzeigeelement 4a angedeutet. Der Zeiger gibt entsprechend der Drehstellung der vierten Scheibeneinheit 4 die Anzahl der Betätigungen an.

Das zuvor beschriebene Zählwerk kann zusammen mit verschiedensten Betätigungseinrichtungen verwendet werden, sofern die Bewegung der Betätigungseinrichtung mit Hilfe eines Betätigungsarmes auf das Zählwerk übertragen wird. Im folgenden wird eine besonders vorteilhafte Betätigungseinrichtung 10 gemäß der Erfindung beispielhaft erläutert.

Die in Fig. 1 und 2 dargestellte Betätigungseinrichtung 10 besteht aus einer zylinderförmigen Hülse 15 und einem zylinderförmigen Unterteil 16. An der Außenseite der Hülse 15 ist ein Betätigungsarm 5 angebracht. Hülse 15 und Unterteil 16 sind über eine bogenförmigen Feder 17 miteinander verbunden. Bei Betätigung der Feder 17, d.h. beim Zusammendrücken von Hülse 15 und Unterteil 16 in axialer Richtung, wird durch geeignete Führungselemente 19, die an der Hülse 15 in Richtung Unterteil 16 weisend angebracht sind, eine geradlinige Bewegung der Hülse 15 bezogen auf das Unterteil 16 gewährleistet. Dabei gleiten die Innenumfangsseite der Hülse 15 auf der Außenseite der Führungselemente 19. Wesentlich ist, daß eine geradlinige Bewegung der Hülse 15 und somit auch eine geradlinige Bewegung des Betätigungsarms 5 beim Zusammendrücken der Betätigungseinrichtung 10 gewährleistet ist, und ein zuverlässiges Eingreifen des Betätigungsarmes 5 in die zahnradförmige erste Scheibeneinheit 1 erzielt wird.

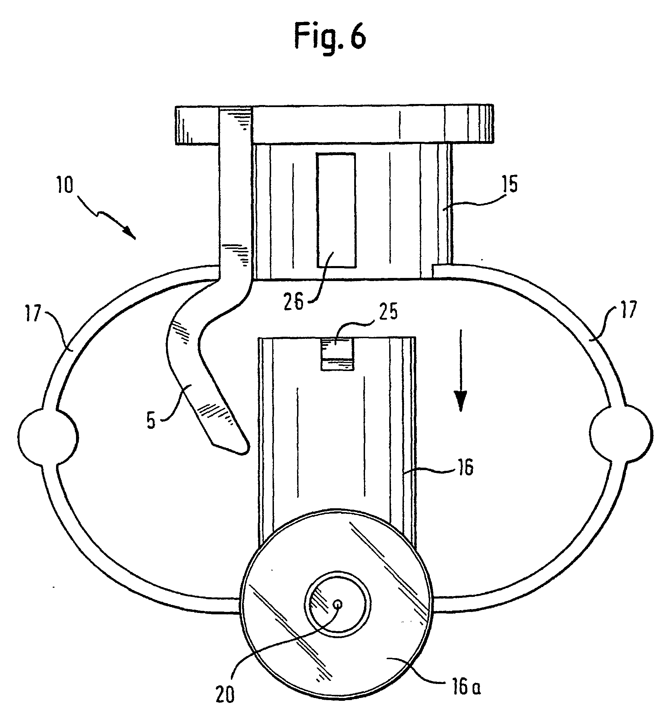

Eine alternative Ausgestaltung der Betätigungseinheit 10 ist in Fig. 6 dargestellt. Fig. 6 zeigt eine Seitenansicht der Betätigungseinheit, betrachtet aus der zu Fig. 1 analogen Blickrichtung. Die in Fig. 6 dargestellte Betätigungseinrichtung 10 weist ebenfalls eine zylinderförmige Hülse 15 und ein Unterteil 16 auf. Im Gegensatz zu der in Fig. 1 bzw. 2 dargestellten Ausführungsform, weist das Unterteil 16 einen weizeren Bereich 16a auf, dessen Längsachse senkrecht zur Längsachse des Unterteils 16 verläuft. Die bohenförmige Feder 17 ist an dem Bereich 16a und an der Hülse 15 befestigt, an der der Betätigungsarm 5 aussen befestigt ist. Das Unterteil 16 dient in diesem Ausführungsbeispiel z.B. zur Aufnahme des Medikamentenbehälters eines herkömmlichen Dosieraerosols oder Inhalators, wie z.'B. eines MDI (metered-dose inhaler), der bei Betätigung eine vorbestimmte Menge an medikamenthaltigem Sprühnebel ausgibt. Die zur Erzeugung des Sprühnebels notwendige Düse ist in das Unterteil 16 bzw. in dessen Bereich 16a integriert, und der erzeugte Sprühnebel wird durch die in Fig. 6 mit Bezugsziffer 20 bezeichnete Düsenöffnung ausgegeben. Somit weist dieses Ausführungsbeispiel den Vorteil auf, daß die zur Erzeugung eines medikamenthaltigen Sprühnebelsnotwendige Düse bereits in der Betätigungseinheit 10 integriert ist. Anzumerken sei ferner, daß die Betätigung der Einheit 10 durch Zusammendrücken der Betätigungs- bzw. Zerstäubereinheit 10 in der in Fig. 6 angezeigten Pfeilrichtung erfolgt. Um die Betätigungseinrichtung inFig. 6 in einen funktionsfähigen Zustand zu bekommen, muß die Hülse soweit in Pfeilrichtung geschoben werden, bis der Rasthaken 25 in das fenster 26 einrastet. Damit sind die Federn 17 vorgespannt, die Hülse 15 befindet sich in Endlagenposition und die Innenseiten der Hülse 15 erhält ihre axiale Führung, indem sie auf der Außenseite des Zylinders 16 gleitet, analog der im Zusammenhang mit Fig. 1 und 2 beschriebenen Führungselemente 19.

Um dem Patienten, der einen derartigen Vernebler anwendet, eine genaue Kontrolle der bereits abgegebenen Dosen bzw. der noch zur erfügung stehenden Sprühstöße zu ermöglichen, ist das zuvor beschriebene Zählwerk über den Lagerbolzen 8 an der Betätigungseinheit 10 befestigt. Durch Zusammendrücken der Betätigungseinrichtung 10, d.h. durch Zusammendrücken der bogenförmigen Federn 17 wird der Betätigungsarm 5, der an der Hülse 15 geeignet angebracht ist, bewegt. Aufgrund der Federwirkung der Federn 17 nimmt die Betätigungseinheit 10 nach dem Zusammendrücken wieder ihre Ausgangsposition ein. Neben dieser Federwirkung kommt den beiden Federn 17 auch eine Führungswirkung im Hinblick auf die geradlinige Bewegung der zylinderförmigen Hülse 15.

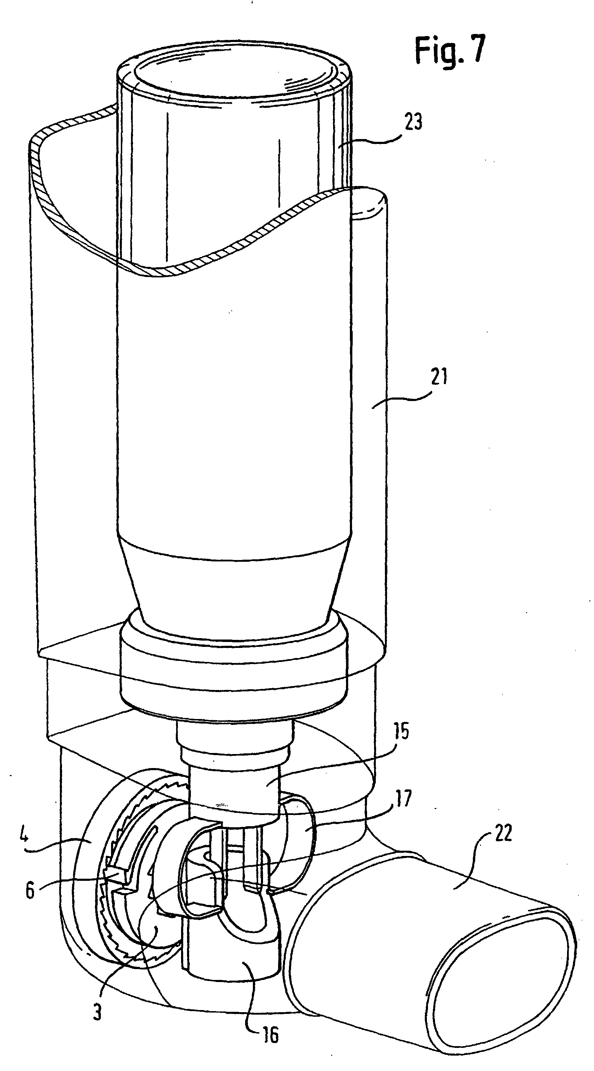

In Fig. 7 ist ein Verwendungsbeispiel dargestellt, bei dem das erfindungsgemäße Zählwerk integriert ist. Fig. 7 zeigt ein Gehäuse 21 einer dosieraerosolausgabevorrichtung, das mit einem Mundstück 22 versehen ist. Das Mundstück 22 ist an dem unteren Bereich des Gehäuses 21 in abgewinkelter Stellung angeordnet. Im oberen Bereich des Gehäuses 21 befindet sich ein Dosieraerosolbehälter 23 zur Aufnahme eines zu zerstäubenden Aerosols. An den unteren Bereich des Aerosolbehälters 23 schließt sich einDüsenbereich an, der in die Führungshülse 15 der Betätigungseinheit 10 eingeführt ist. Die Betätigunhseinheit 10 ist mit dem erfindungsgemäßen Zählwerk, bestehend aus den Scheibeneinheiten 1, 2, 3 und 4, verbunden, um die abgegebenen bzw. noch verbleibenden Dosierungen zu zählen und anzuzeigen. Bei jedem Pumpstoß, der wie zuvor beschrieben, durch die Betätigung der Betätigungseinheit 10 ausgelöst wird, wird eine bestimmte Aerosolmenge ausgegeben und über eine entsprechende Düse zerstäubt, deren Aufbau und Verwendung mit derartigen Dosieraerosolvorrichtungen bekannt ist, und kann vom Patienten über das Mundstück 22 eingeatmet werden.

Vorteilhaft ist, daß für den Einsatz eines erfindungsgemäßen Zählwerks bei einem herkömmlichen Vernebler bzw. Inhalator dieser nicht in seinen wesentlichen Komponenten, insbesondere nicht die Düse, verändert werden muß. Durch eine einfache Anbringung eines Betätigungsarms zum Betätigen des Zählwerks, wird ein einfacher und unkomplizierter Aufbau ermöglicht, der universell mit bereits bekannten Verneblern und Inhalatoren verwendet werden kann. Auf diese Weise kann auf eine aufwendige Modifikation der bereits bekannten Vernebler verzichtet werden. Dies ist wesentlich von Vorteil, da beispielsweise bei einer Änderung der Düsenform derartige Inhaltoren erneut medizinische Prüfungen zur Erlangung der Zulassung durchlaufen müssen, die in der Regel langwierig, aufwendig und somit kostspielig sind.

## Patentansprüche

1. Zählwerk mit vier Scheibeneinheiten (1, 2, 3, 4), die parallel zueinander angeordnet sind, deren Mittelpunkte auf einer zu den Ebenen der vier Scheibeneinheiten (1, 2, 3, 4) senkrecht stehenden Achse liegen und von denen eine erste Scheibeneinheit (1) erste Aufnahmemittel (1a) zur Aufnahme einer eine Drehbewegung der ersten Scheibeneinheit um die Achse bewirkenden Kraft und erste Übertragungsmittel zur Übertragung der Drehbewegung auf eine zweite Scheibeneinheit (2) aufweist, die zweite Aufnahmemittel zur Aufnahme einer durch die ersten Übertragungsmittel übertragenen und eine Drehbewegung der zweiten Scheibeneinheit (2) um die Achse bewirkenden Kraft und einen Federnocken (6), der an der Außenkontur der zweiten Scheibeneinheit (2) federnd angeordnet ist, zur Übertragung der Drehbewegung auf eine vierte Scheibeneinheit (4) aufweist, die eine Innenverzahnung (13) zur Aufnahme einer durch den Federnocken (6, 6a) übertragenen und eine Drehbewegung der vierten Scheibeneinheit (4) um die Achse bewirkenden Kraft aufweist, wobei an der Außenkontur einer dritten Scheibeneinheit (3) mindestens ein Vorsprung (11) vorgesehen ist, der bei einer vorbestimmten Drehstellung der zweiten Scheibeneinheit (2) in Bezug auf die dritte Scheibeneinheit (3) den Federnocken (6) in radialer Richtung auslenkt, so dass der Federnocken (6) in Eingriff mit der Innenverzahnung (13) der vierten Scheibeneinheit (4) gerät und wobei mindestens ein Zahn der Innenverzahnung (13) der vierten Scheibeneinheit (4) ausgefüllt ist, um einen Verriegelungseffekt zu bewirken.

2. Zählwerk mit vier Scheibeneinheiten (1, 2, 3, 4), die parallel zueinander angeordnet sind, deren Mittelpunkte auf einer zu den Ebenen der viert Scheibeneinheiten (1, 2, 3, 4) senkrecht stehenden Achse liegen und von denen eine erste Scheibeneinheit (1) erste Aufnahmemittel (1a) zur Aufnahme einer eine Drehbewegung der ersten Scheibeneinheit um die Achse bewirkenden Kraft und erste Übertragungsmittel zur Übertragung der Drehbewegung auf eine zweite Scheibeneinheit (2) aufweist, die zweite Aufnahmemittel zur Aufnahme einer durch die ersten Übertragungsmittel übertragenen und eine Drehbewegung der zweiten Scheibeneinheit (2) um die Achse bewirkenden Kraft und einen Federnocken (6), der an der Außenkontur der zweiten Scheibeneinheit (2) federnd angeordnet ist, zur Übertragung der Drehbewegung auf eine vierte Scheibeneinheit (4) aufweist, die eine Innenverzahnung (13) zur Aufnahme einer durch den Federnocken (6, 6a) übertragenen und eine Drehbewegung der vierten Scheibeneinheit (4) um die Achse bewirkenden Kraft aufweist, wobei an der Außenkontur einer dritten Scheibeneinheit (3) mindestens ein Vorsprung (11) vorgesehen ist, der bei einer vorbestimmten Drehstellung der zweiten Scheibeneinheit (2) in Bezug auf die dritte Scheibeneinheit (3) den Federnocken (6) in radialer Richtung auslenkt, so dass der Federnocken (6) in Eingriff mit der Innenverzahnung (13) und der vierten Scheibeneinheit (4) gerät und wobei mindestens ein Zahn der Innenverzahnung (13) der vierten Scheibeneinheit (4) entfernt ist, um einen Eingriff des Federnockens (6) zu unterdrücken.

3. Zählwerk nach Anspruch 1 oder 2,
**dadurch gekennzeichnet, dass**
der Federnocken (6) keilförmig ist und dass die Breite des Federnockens (6) grösser ist als die Dicke der zweiten Scheibeneinheit (2), so dass der Federnocken (6) die zweite Scheibeneinheit (2) in Richtung der Achse überragt.

4. Zählwerk nach Anspruch 1, 2 oder 3,
**dadurch gekennzeichnet, dass**
die dritte Scheibeneinheit (3) eine feste Position definiert und die erste, zweite und vierte Scheibeneinheit (1, 2, 4) bezüglich der dritten Scheibeneinheit (3) drehbar gelagert sind.

5. Zählwerk nach einem der Ansprüche 1 bis 4,
**dadurch gekennzeichnet, dass**
die ersten Aufnahmemittel der ersten Scheibeneinheit (1) eine Außenverzahnung (1a) ist.

6. Zählwerk nach einem der Ansprüche 1 bis 4,
**dadurch gekennzeichnet, dass**
die ersten Übertragungsmittel und die zweiten Aufnahmemittel in Form einer festen Verbindung zwischen der ersten und der zweiten Scheibeneinheit (1, 2) realisiert sind.

7. Zählwerk nach einem der Ansprüche 1 bis 6,
**dadurch gekennzeichnet, dass**
die vierte Scheibeneinheit (4) eine um den Mittelpunkt der vierten Scheibeneinheit (4) kreisförmig ausgebildete zweite Verzahnung (14) aufweist und die dritte Scheibeneinheit (3) eine Vielzahl von Rastverzahnungen (12) für den Eingriff in die zweite Verzahnung (14) der vierten Scheibeneinheit (4) aufweist.

8. Zählwerk nach einem der Ansprüche 1 bis 7,
**dadurch gekennzeichnet, dass**
der Radius der zweiten Scheibeneinheit (2) größer ist als der der ersten Scheibeneinheit (1), der Radius der dritten Scheibeneinheit (3) größer ist als der der ersten Scheibeneinheit (1) und kleiner ist, als der der zweiten Scheibeneinheit (2), und der Radius der vierten Scheibeneinheit (4) größer ist als der der ersten, zweiten und dritten Scheibeneinheit (1, 2, 3).

9. Zählwerk nach einem der Ansprüche 1 bis 8,
**dadurch gekennzeichnet, dass**
die vier Scheibeneinheiten (1, 2, 3, 4) mittels eines Lagerbolzens (8) gelagert sind, der einen ersten Abschnitt (8b) für die gleitende Lagerung der ersten und zweiten Scheibeneinheit (1, 2) und einen zweiten Abschnitt (8a) für die gleitende Lagerung der vierten Scheibeneinheit (4) aufweist und an dem die dritte Scheibeneinheit (3) zwischen dem ersten und zweiten Abschnitt (8b, 8a) befestigt ist.

10. Zählwerk nach Anspruch 9,
**dadurch gekennzeichnet, dass**
der Lagerbolzen (8) zur Befestigung des Zählwerks einen dritten Abschnitt (8c) aufweist, der sich auf der Seite der ersten Scheibeneinheit (1) aus dem Zählwerk heraus erstreckt.

11. Zählwerk nach Anspruch 9 oder 10,
**dadurch gekennzeichnet, dass**
die zweite Scheibeneinheit (2) eine Innenverzahnung (7) aufweist und an dem Lagerbolzen (8) Verriegelungselemente (9) befestigt sind, die mit der Innenverzahnung (7) der zweiten Scheibeneinheit (2) in Wechselwirkung stehen.

12. Zählwerk nach einem der vorangegangenen Ansprüche,
**dadurch gekennzeichnet, dass**
es mit einer Betätigungseinrichtung (10) verbunden ist.

13. Zählwerk nach Anspruch 12,
**dadurch gekennzeichnet, dass**
die Betätigungseinrichtung (10) eine Hülse (15) und ein Unterteil (16) umfasst.

14. Zählwerk nach Anspruch 12 oder 13,
**dadurch gekennzeichnet, dass** an der Hülse (15) ein Betätigungsarm (5) angebracht ist.

15. Zählwerk nach einem der Ansprüche 12 bis 14,
**dadurch gekennzeichnet, dass**
die Hülse (15) und das Unterteil (16) der Betätigungseinheit (10) über Federelemente (17) miteinander verbunden sind.

16. Zählwerk nach einem der Ansprüche 12 bis 15,
**dadurch gekennzeichnet, dass**
an der Hülse (15) in Richtung Unterteil (16) weisend Führungselemente (19) angebracht sind.

17. Zählwerk nach einem der Ansprüche 12 bis 16,
**dadurch gekennzeichnet, dass** die Innenumfangsseite der Hülse (15) entlang der Außenseiten der Führungselemente (19) gleitet.

18. Zählwerk nach einem der Ansprüche 12 oder 13,
**dadurch gekennzeichnet, dass**
das Unterteil (16) einen Bereich (16a) aufweist, dessen Längsachse senkrecht zur Längsachse des Unterteils (16) verläuft.

19. Zählwerk nach Anspruch 18,
**dadurch gekennzeichnet, dass**
die Hülse (15) und der Bereich (16a) über Federelemente (17) miteinander verbunden sind.

20. Zählwerk nach einem der Ansprüche 18 oder 19,
**dadurch gekennzeichnet, dass**
die Hülse (15) ein Fenster (26) aufweist und an dem Unterteil (16) ein Rasthaken (25) derart angebracht ist, dass der Rasthaken (25) zur axialen Führung der Hülse (15) in das Fenster (16) einrastet.

21. Verwendung eines Zählwerks nach einem der vorangegangenen Ansprüche in Inhalatoren, Verneblern oder ähnlichen Dosieraerosolvorrichtungen.

## Claims

1. Meter with four disc units (1, 2, 3, 4) which are arranged parallel to each other, whose centres lie on an axis perpendicular to the planes of the four disc units (1, 2, 3, 4) and of which a first disc unit (1) comprises first receiving means (1a) for receiving a force which causes a rotational movement of the first disc unit about the axis and first transmitting means for transmission of the rotational movement to a second disc unit (2), the second receiving means for receiving a force which is transmitted by the first transmitting means and causes a rotational movement of the second disc unit (2) about the axis, and a spring cam (6) which is spring-mounted on the outer contour of the second disc unit (2), for transmission of the rotational movement to a fourth disc unit (4) which comprises an internal tooth system (13) for receiving a force which is transmitted by the spring cam (6, 6a) and causes a rotational movement of the fourth disc unit (4) about the axis, wherein on the outer contour of a third disc unit (3) is provided at least one projection (11) which, in a predetermined rotational position of the second disc unit (2) in relation to the third disc unit (3), deflects the spring cam (6) in a radial direction so that the spring cam (6) engages with the internal tooth system (13) of the fourth disc unit (4), and wherein at least one tooth of the internal tooth system (13) of the fourth disc unit (4) is filled out to cause a locking effect.

2. Meter with four disc units (1, 2, 3, 4) which are arranged parallel to each other, whose centres lie on an axis perpendicular to the planes of the four disc units (1, 2, 3, 4) and of which a first disc unit (1) comprises first receiving means (1a) for receiving a force which causes a rotational movement of the first disc unit about the axis and first transmitting means for transmission of the rotational movement to a second disc unit (2), the second receiving means for receiving a force which is transmitted by the first transmitting means and causes a rotational movement of the second disc unit (2) about the axis, and a spring cam (6) which is spring-mounted on the outer contour of the second disc unit (2), for transmission of the rotational movement to a fourth disc unit (4) which comprises an internal tooth system (13) for receiving a force which is transmitted by the spring cam (6, 6a) and causes a rotational movement of the fourth disc unit (4) about the axis, wherein on the outer contour of a third disc unit (3) is provided at least one projection (11) which, in a predetermined rotational position of the second disc unit (2) in relation to the third disc unit (3), deflects the spring cam (6) in a radial direction so that the spring cam (6) engages with the internal tooth system (13) of the fourth disc unit (4), and wherein at least one tooth of the internal tooth system (13) of the fourth disc unit (4) is removed to prevent engagement of the spring cam (6).

3. Meter according to claim 1 or 2, **characterised in that** the spring cam (6) is wedge-shaped and **in that** the width of the spring cam (6) is greater than the thickness of the second disc unit (2), so that the spring cam (6) protrudes beyond the second disc unit (2) in the direction of the axis.

4. Meter according to claim 1, 2 or 3, **characterised in that** the third disc unit (3) defines a fixed position and the first, second and fourth disc units (1, 2, 4) are mounted rotatably relative to the third disc unit (3).

5. Meter according to any of claims 1 to 4, **characterised in that** the first receiving means of the first disc unit (1) is an external tooth system (1a).

6. Meter according to any of claims 1 to 4, **characterised in that** the first transmitting means and the second receiving means are constructed in the form of a rigid connection between the first and second disc units (1, 2).

7. Meter according to any of claims 1 to 6, **characterised in that** the fourth disc unit (4) comprises a second tooth system (14) designed as a circle round the centre of the fourth disc unit (4), and the third disc unit (3) comprises a plurality of latch tooth systems (12) for engagement in the second tooth system (14) of the fourth disc unit (4).

8. Meter according to any of claims 1 to 7, **characterised in that** the radius of the second disc unit (2) is greater than that of the first disc unit (1), the radius of the third disc unit (3) is greater than that of the first disc unit (1) and smaller than that of the second disc unit (2), and the radius of the fourth disc unit (4) is greater than that of the first, second and third disc units (1, 2, 3).

9. Meter according to any of claims 1 to 8, **characterised in that** the four disc units (1, 2, 3, 4) are mounted by means of a bearing pin (8) which comprises a first section (8b) for sliding bearing of the first and second disc units (1, 2) and a second section (8a) for sliding bearing of the fourth disc unit (4) and to which the third disc unit (3) is attached between the first and second sections (8b, 8a).

10. Meter according to claim 9, **characterised in that** for attachment of the meter the bearing pin (8) comprises a third section (8c) which extends out of the meter on the side of the first disc unit (1).

11. Meter according to claim 9 or 10, **characterised in that** the second disc unit (2) comprises an internal tooth system (7) and attached to the bearing pin (8) are locking elements (9) which interact with the internal tooth system (7) of the second disc unit (2).

12. Meter according to any of the preceding claims, **characterised in that** it is connected to an actuator (10).

13. Meter according to claim 12, **characterised in that** the actuator (10) includes a sleeve (15) and a lower portion (16).

14. Meter according to claim 12 or 13, **characterised in that** on the sleeve (15) is mounted an actuating arm (5).

15. Meter according to any of claims 12 to 14, **characterised in that** the sleeve (15) and the lower portion (16) of the actuating unit (10) are connected to each other by spring elements (17).

16. Meter according to any of claims 12 to 15, **characterised in that** on the sleeve (15) are mounted guide elements (19) pointing in the direction of the lower portion (16).

17. Meter according to any of claims 12 to 16, **characterised in that** the inner circumferential side of the sleeve (15) slides along the outer sides of the guide elements (19).

18. Meter according to either of claims 12 or 13, **characterised in that** the lower portion (16) comprises a region (16a) whose longitudinal axis runs perpendicularly to the longitudinal axis of the lower portion (16).

19. Meter according to claim 18, **characterised in that** the sleeve (15) and the region (16a) are connected to each other by spring elements (17).

20. Meter according to either of claims 18 or 19, **characterised in that** the sleeve (15) comprises a window (26), and a latch hook (25) is mounted on the lower portion (16) in such a way that the latch hook (25) latches in the window (16) for axially guiding the sleeve (15).

21. Use of a meter according to any of the preceding claims in inhalers, nebulisers or the like aerosol metering devices.

## Revendications

1. Dispositif compteur muni de quatre unité à disque (1, 2, 3, 4) disposées parallèlement les unes les autres, dont les centres sont situés sur un axe perpendiculaire aux plans des quatre unités à disque (1, 2, 3, 4) et dont une première unité à disque (1) présente des premiers moyens support (1a), pour supporter une force provoquant un mouvement rotatif de la première unité à disque autour de l'axe, et un premier moyen de transmission, pour transmettre le mouvement rotatif à une deuxième unité à disque (2), qui présente des deuxièmes moyens support, pour supporter une force transmise par le premier moyen de transmission et provoquant un mouvement rotatif de la deuxième unité à disque (2) autour de l'axe, et présente un ergot élastique (6), monté élastiquement sur le contour extérieur de la deuxième unité à disque (2) pour assurer la transmission du mouvement rotatif à une quatrième unité à disque (4), présentant une denture intérieure (13) pour supporter une force transmise par l'ergot élastique (6, 6a) et provoquant un mouvement rotatif de la quatrième unité à disque (4) autour de l'axe, où, sur le contour extérieur d'une troisième unité à disque (3), est prévue au moins une saillie (11) qui, en une position de rotation prédéterminée de la deuxième unité à disque (2) par rapport à la troisième unité à disque (3), dévie l'ergot élastique (6) en direction radiale, de manière que l'ergot élastique (6) vienne en prise avec la denture intérieure (13) de la quatrième unité à disque (4), et où au moins une dent de la denture intérieure (13) de la quatrième unité à disque (4) est remplie pour provoquer un effet de verrouillage.

2. Dispositif compteur muni de quatre unité à disque (1, 2, 3, 4) disposées parallèlement les unes les autres, dont les centres sont situés sur un axe perpendiculaire aux plans des quatre unités à disque (1, 2, 3, 4) et dont une première unité à disque (1) présente des premiers moyens support (1a), pour supporter une force provoquant un mouvement rotatif de la première unité à disque autour de l'axe, et un premier moyen de transmission, pour transmettre le mouvement rotatif à une deuxième unité à disque (2), qui présente des deuxièmes moyens support, pour supporter une force transmise par le premier moyen de transmission et provoquant un mouvement rotatif de la deuxième unité à disque (2) autour de l'axe, et présente un ergot élastique (6), monté élastiquement sur le contour extérieur de la deuxième unité à disque (2) pour assurer la transmission du mouvement rotatif à une quatrième unité à disque (4), présentant une denture intérieure (13) pour supporter une force transmise par l'ergot élastique (6, 6a) et provoquant un mouvement rotatif de la quatrième unité à disque (4) autour de l'axe, où, sur le contour extérieur d'une troisième unité à disque (3), est prévue au moins une saillie (11) qui, en une position de rotation prédéterminée de la deuxième unité à disque (2) par rapport à la troisième unité à disque (3), dévie l'ergot élastique (6) en direction radiale, de manière que l'ergot élastique (6) vienne en prise avec la denture intérieure (13) de la quatrième unité à disque (4), et où au moins une dent de la denture intérieure (13) de la quatrième unité à disque (4) est enlevée pour faire cesser la mise en prise de la came élastique (6).

3. Dispositif compteur selon la revendication 1 ou 2, **caractérisé en ce que** l'ergot élastique (6) est cunéiforme, et **en ce que** la largeur de l'ergot élastique (6) est supérieure à l'épaisseur de la deuxième unité à disque (2), de manière que l'ergot élastique (6) dépasse de la deuxième unité à disque (2) dans la direction de l'axe.

4. Dispositif compteur selon la revendication 1, 2 ou 3, **caractérisé en ce que** la troisième unité à disque (3) définit une position fixe et la première, la deuxième et la quatrième unité à disque (1, 2, 4) sont montées à rotation par rapport à la troisième unité à disque.

5. Dispositif compteur selon l'une des revendications 1 à 4, **caractérisé en ce que** les premiers moyens supports de la première unité à disque (1) sont formés d'une denture extérieure (1a).

6. Dispositif compteur selon l'une des revendications 1 à 4, **caractérisé en ce que** les premiers moyens de transmission et les deuxièmes moyens supports sont réalisés sous la forme d'une liaison rigide entre la première et la deuxième unité à disque (1, 2).

7. Dispositif compteur selon l'une des revendications 1 à 6, **caractérisé en ce que** la quatrième unité à disque (4) présente une deuxième denture (14) en forme de cercle, réalisée autour du centre de la quatrième unité à disque (4), et la troisième unité à disque (3) présente une pluralité de dents d'encliquetage (12) pour l'engrènement dans la deuxième denture (14) de la quatrième unité à disque (4).

8. Dispositif compteur selon l'une des revendications 1 à 7, **caractérisé en ce que** le rayon de la deuxième unité à disque (2) est supérieur à celui de la première unité à disque (1), le rayon de la troisième unité à disque (2) est supérieur à celui de la première unité à disque (1) et inférieur à celui de la deuxième unité à disque (2), et le rayon de la quatrième unité à disque (4) est supérieur à ceux de la première, de la deuxième et de la troisième unité à disque (1, 2, 3).

9. Dispositif compteur selon l'une des revendications 1 à 8, **caractérisé en ce que** les quatre unités à disque (1, 2, 3, 4) sont montées à l'aide d'un boulon formant palier (8), présentant un premier tronçon (8b) pour le montage en palier glissant de la première et de la deuxième unité à disque (1, 2), et d'un deuxième tronçon (8a) pour le montage en palier glissant de la quatrième unité à disque (4), et auquel est fixée la troisième unité à disque (3), entre le premier et le deuxième tronçon (8b, 8a).

10. Dispositif compteur selon la revendication 9, **caractérisé en ce que** le boulon formant palier (8) présente, pour assurer la fixation du dispositif compteur, un troisième tronçon (8c) s'étendant sur le côté de la première unité à disque (1) en ressortant du dispositif compteur.

11. Dispositif compteur selon la revendication 9 ou 10, **caractérisé en ce que** la deuxième unité à disque (2) présente une denture intérieure (7) et, sur le boulon formant palier (8), sont fixés des éléments de verrouillage (9) qui sont en interaction avec la denture intérieure (7) de la deuxième unité à disque (2).

12. Dispositif compteur selon l'une des revendications précédentes, **caractérisé en ce qu'**il est relié à un dispositif d'actionnement (10).

13. Dispositif compteur selon la revendication 12, **caractérisé en ce que** le dispositif d'actionnement (10) comprend une douille (15) et une partie inférieure (16).

14. Dispositif compteur selon la revendication 12 ou 13, **caractérisé en ce qu'**un bras d'actionnement (5) est monté sur la douille (15).

15. Dispositif compteur selon l'une des revendications 12 à 14, **caractérisé en ce que** la douille (15) et la partie inférieure (16) de l'unité d'actionnement (10) sont reliées ensemble par des éléments élastiques (17).

16. Dispositif compteur selon l'une des revendications 12 à 15, **caractérisé en ce que**, sur la douille (15), sont montés des éléments de guidage (19) tournés dans la direction de la partie inférieure (16).

17. Dispositif compteur selon l'une des revendications 12 à 16, **caractérisé en ce que** la face périphérique intérieure de la douille (15) coulisse le long des faces extérieures des éléments de guidage (19).

18. Dispositif compteur selon l'une des revendications 12 ou 13, **caractérisé en ce que** la partie inférieure (16) présente une zone (16a) dont l'axe longitudinal s'étend perpendiculairement par rapport à l'axe longitudinal de la partie inférieure (16).

19. Dispositif compteur selon la revendication 18, **caractérisé en ce que** la douille (15) et la zone (16a) sont reliées ensemble par des éléments élastiques (17).

20. Dispositif compteur selon l'une des revendications 18 ou 19, **caractérisé en ce que** la douille (15) présente une fenêtre (26) et, sur la partie inférieure (16), un crochet d'encliquetage (25) est monté, de manière que le crochet d'encliquetage (25) s'encliquète dans la fenêtre (16) pour obtenir un guidage axial de la douille (15).

21. Utilisation d'un dispositif compteur selon l'une des revendications précédentes, dans des inhalateurs, des nébulisations, ou bien des dispositifs à aérosol doseurs analogues.
